# EUROPÄISCHE PATENTSCHRIFT

(11) **EP 4 351 448 B1**
(45) Veröffentlichungstag und Bekanntmachung des Hinweises auf die Patenterteilung: **04.09.2024**
(21) Anmeldenummer: 22757494.4
(22) Anmeldetag: 20.07.2022
(51) Int. Cl.: A61B 17/29, A61B 17/16, A61B 17/32

(54) **ABWINKELBARER SCHAFT FÜR EIN MEDIZINISCHES HANDINSTRUMENT**
BENDABLE SHAFT FOR A MEDICAL HAND-HELD INSTRUMENT
TIGE PLIABLE POUR UN INSTRUMENT MÉDICAL À MAIN

(30) Priorität: 27.07.2021 DE 102021119386
(43) Veröffentlichungstag der Anmeldung: 17.04.2024
(73) Patentinhaber: Aesculap AG, 78532 Tuttlingen (DE)
(72) Erfinder: HAGEN, Thomas, 78532 Tuttlingen (DE); HERMLE, Simone, 78050 Villingen-Schwenningen (DE); PFISTER, Ralf, 78647 Trossingen (DE); HAHN, Lukas, 72488 Sigmaringen (DE); BÜRK, André, 78056 Villingen-Schwenningen (DE)
(74) Vertreter: Winter, Brandl - Partnerschaft mbB
(86) Internationale Anmeldenummer: PCT/EP2022/070333
(87) Internationale Veröffentlichungsnummer: WO 2023/006541

(56) Entgegenhaltungen:
- WO-A1-99/21686
- DE-T5- 10 392 849
- RU-C2- 2 181 566
- US-A- 5 575 799
- US-A- 6 050 989

## Beschreibung

### Technisches Gebiet

Die vorliegende Offenbarung betrifft einen Schaft eines oder für ein medizinisches Handinstrument.

In der Operationstechnik ist es vorteilhaft, den distalen Schaftabschnitt eines Schafts von einem medizinischen Handinstrument abzuwinkeln. Dadurch können Operationen in geringem Raum durchgeführt werden, beispielsweise bei Operationen an der Wirbelsäule.

### Stand der Technik

Aus dem Bereich der Operationsroboter sind schon seit längerem Instrumente bekannt, die eine Abwinklung der distalen Spitze ermöglichen. Dadurch wird eine präzise Bewegung der Instrumente auf engstem Raum ermöglicht. Allerdings werden bei diesen Instrumenten keine rotierenden Werkzeuge abgewinkelt. Ein Beispiel hierfür ist der Operationsroboter "Da Vinci" von der Firma Intuitive Surgical.

Es sind verschiedene abwinkelbare medizinische Geräte auf dem Markt erhältlich. So hat die Firma Human Xtensions eine abwinkelbare Zange entwickelt. Ein Operateur kann handgehaltene robotische Instrumente nutzen, um "grobe" Handbewegungen in feinfühlige Bewegungen an der Instrumentenspitze zu übersetzen. Eine Instrumentenspitze kann bei diesem Instrument über einen flexiblen Bereich, welcher sich über eine Länge von ca. 20 mm erstreckt, abgewinkelt werden. Der flexible Bereich wird durch eine Art Kunststoff-Stent gestützt. Die Verstellung erfolgt mittels außen am Stent vorbei geführter Drahtlitzen. Nachteilig ist hier die Länge des Biegebereichs, sowie die sehr nachgiebige Spitze. Die Nachgiebigkeit ist hierbei durch den Kunststoff-Stent und die Drahtlitzen bedingt.

Weiterhin gibt es bereits Hersteller von winkelbaren Fräs-Handstücken/ medizinischen Handinstrumenten. Diese werden vorwiegend bei endoskopischen Eingriffen an der Wirbelsäule eingesetzt und ermöglichen minimal-invasive Techniken, sowie die einfache Bearbeitung schwer zugänglicher Strukturen in diesem Bereich. Die Gelenkkonstruktionen dieser Fräs-Handstücke weisen eine eher offene Bauform auf und besitzen eine gewisse Flexibilität der Winkelung. D.h. bei Druck auf die Fräserspitze wird der Winkel leicht verändert. Weiterhin sind bei diesen Fräs-Handstücken nur Abwinkelungen von bis zu 36° möglich. Ein Beispiel hierfür sind Fräs-Handstücke der Firma Joimax.

Es existieren winkelbare Akkuschrauber mit einer schrägen Ebene. Im Bereich der Heimwerker-Geräte gibt es Akkuschrauber mit winkelbarem Kopf. Diese weisen ein ähnliches Drehgelenk wie die nachfolgend beschriebene Offenbarung auf und besitzen eine sehr gute Kraftaufnahme der Winkelung (starres Gelenk). Je nach Winkelung der Drehebene sind hier Abwinkelungen von bis zur 90° möglich. Jedoch müssen die Akkuschrauber von außen verstellt werden. Sie verfügen somit nicht über die interne Ansteuerung.

Ferner existieren Highspeed Fräshandstücke/ medizinische Handinstrumente mit gewinkeltem Schaft. Dank wechselbarer Schäfte kann ein Handstück zwischen drei Varianten gewählt werden: 0°-, 7,5°- und 15°-Abwinkelung. Größter Nachteil ist dabei der große Biegeradius, über den die Abwinkelung realisiert wird. Dieser verbraucht sehr viel Platz und beschränkt die Aktionsmöglichkeiten im OP-Feld. Außerdem ist der Winkel nicht intraoperativ verstellbar. Durch die fixe Winkelung ist kein endoskopisches Arbeiten mit den gewinkelten Schäften möglich, da sie sich nicht in den geraden Arbeitskanal des Endoskops einschieben lassen. Weiterhin ist die maximale Abwinkelung mit 15° nicht besonders groß.

Aus den Offenbarungen DE 10 2017 010 033 A1 und US 10 178 998 B2 sind Fräser mit abwinkelbaren Köpfen bekannt. Beide Lösungen sind über Gabelgelenke realisiert.

Aus der DE 103 92 849 T5 ist ein Fräser mit einem distalen und einem proximalen Schaftabschnitt bekannt, die jeweils derart zueinander verdrehbar sind, dass die beiden Schaftabschnitte durch ihre angestellten Stirnseiten zueinander abgewinkelbar sind. Die beiden Schaftabschnitte werden dabei durch ein Kegelradgetriebe gegeneinander abgewinkelt.

Die RU 2 181 566 C2 offenbart ein abwinkelbares Handinstrument. Dabei weist ein rohrförmiger Zwischenabschnitt eine Verzahnung an jedem Ende des Zwischenabschnitts auf. Die Verzahnungen kämmen mit einer entsprechenden Verzahnung an einem distalen und einem proximalen Schaftabschnitt, wobei der distale Schaftabschnitt durch Drehung um eine Längsachse relativ zum proximalen Schaftabschnitt abwinkelbar ist. Der distale und der proximale Schaftabschnitt haben jeweils angestellte Stirnseiten, durch die die Schaftabschnitte abwinkelbar sind.

Die US 6 050 989 A zeigt ein chirurgisches Handstück mit einer abwinkelbaren distalen Buche. Die distale Buchse ist relativ zu einer proximalen Buchse rotierbar, wobei die distale und die proximale Buchse über ein federvorgespanntes Verbindungselement miteinander verbunden sind. US 5 575 799 A zeigt ferner ein chirurgisches Handstück mit einer abwinkelbaren distalen Spitze. Die distale Spitze ist dabei durch eine Kegelraderzahnung gegenüber einem proximalen Schaftabschnitt rotierbar und durch angestellte Stirnseiten abwinkelbar.

Aus der WO 99 / 21 686 A1 ist ferner ein Bohrfutter für eine Handbohrmaschine mit einem abwinkelbaren Bohrer bekannt.

### Zusammenfassung der Offenbarung

Die Aufgabe der Offenbarung besteht deshalb darin, die Nachteile des Stands der Technik zu überwinden und einen Schaft für ein medizinisches Handinstrument bereitzustellen, bei dem ein distaler Schaftabschnitt im Betrieb abgewinkelt werden kann und sich der Winkel zwischen dem distalen Schaftabschnitt und einem proximalen Schaftabschnitt auch unter Belastung nicht ändert. Insbesondere soll der Schaft des Handinstruments durch einen einfachen und fehlerresistenten Mechanismus abwinkelbar sein.

Diese Aufgabe wird offenbarungsgemäß durch einen Schaft für ein medizinisches Handinstrument mit den Merkmalen des Anspruchs 1 gelöst. Vorteilhafte Weiterbildungen der Offenbarung sind Gegenstand der beigefügten Unteransprüche.

Die Offenbarung betrifft demzufolge einen Schaft für ein medizinisches Handinstrument mit einem distalen Schaftabschnitt und einem proximalen Schaftabschnitt, deren jeweils einander zugewandte Stirnseiten, von denen zumindest eine mit einem Anstellwinkel ungleich 90° bezüglich der jeweiligen Schaftlängsachse angestellt ist, sodass sich je nach Relativdrehposition der beiden Schaftabschnitte unterschiedliche Schaftformen ergeben. Das Ritzel ist durch ein flexibles Übertragungselement mit der Verstellbuchse in dem distalen Schaftabschnitt rotationsübertragend verbunden.

Das flexible Übertragungselement kann dabei vorzugsweise eine Drehwelle oder ein Blech(-streifen) sein, das sich sowohl konzentrisch oder auch eiernd (d.h. auf einer Umlaufbahn) um eine Längsachse dreht. Das Ritzel ist mit dem flexiblen Übertragungselement verbunden. Die Verbindung kann beispielsweise durch Schweißen und/oder Kleben oder eine andere lösbare oder nicht lösbare Fügetechnik realisiert sein. Auf der dem Ritzel abgewandten Seite des flexiblen Übertragungselements kann bevorzugt die Verstellbuchse angebracht sein. Auch diese Verbindung kann durch Schweißen oder Kleben realisiert sein. Das flexible Übertragungselement übertragt die Rotation/ Drehung des Ritzels auf die Verstellbuchse.

In anderen Worten hat der Schaft einen abwinkelbaren distalen Schaftabschnitt. Der distale Schaftabschnitt und der proximale Schaftabschnitt weisen beide jeweils ein schräges Ende/ eine bezüglich der jeweiligen Schaftabschnittsachse angestellte Stirnseite auf. D.h. jeweils ein Ende/ Endabschnitt/ Stirnseite des distalen und proximalen Schaftabschnitts ist nicht gerade, sondern abgeschrägt/ angestellt. Die Schrägungen/ angestellten Endabschnitte/ Stirnseiten haben jeweils den im Wesentlichen gleichen Anstellwinkel. Darum passen die Schrägungen derart zueinander, dass der proximale und der distale Schaftabschnitt in einer bestimmten Relativdrehposition einen geraden Schaft/ ein gerades Rohr bilden. Wenn nun der distale Schaftabschnitt um seine Längsachse relativ zum proximalen Schaftabschnitt rotiert und der proximale Schaftabschnitt stehen bleibt, wird der distale Schaftabschnitt durch die angestellten Stirnseiten/ Endabschnitte zwangsläufig abgewinkelt.

Die vorstehend beschriebene Lösung weist folgende Vorteile auf:
- Der distale Schaftabschnitt des Schafts kann stufenlos verstellt werden.
- Die Ansteuerung der Drehmechanik ist dabei vollständig in den Schaft integriert und lässt sich über eine nicht näher beschriebene Drehhülse am proximalen Endabschnitt des Schaftes verstellen.
- Das speziell aufgebaute Drehgelenk ist sehr stabil, leichtgängig und vollkommen unempfindlich gegenüber äußeren Biegekräften. Dadurch wird eine sehr genaue Position des distalen Schaftabschnitts ermöglicht, die sich auch nicht durch auftretende Fräskräfte verändert. Dies ist ein entscheidender Vorteil bei der Anwendung von robotergesteuerten Techniken.
- Eine hohe Präzision und ein geringes Fehlerrisiko sind hier die wichtigsten Argumente.

Der distale Schaftabschnitt des medizinischen Handinstruments ist im Betrieb abwinkelbar. Das heißt es ist möglich, den distalen Schaftabschnitt während einer Operation abzuknicken. Dadurch, dass der distale Schaftabschnitt auf der Schrägung/ angestellten Stirnseite des proximalen Schaftabschnitts aufliegt, ist der distale Schaftabschnitt fest gelagert.

Das medizinische Handinstrument weist bevorzugt ein Einstellrad, den (angestellten) proximalen Schaftabschnitt, den relativ dazu abwinkelbaren distalen Schaftabschnitt, einen flexiblen Fräser und ein Lager auf. Der proximale Schaftabschnitt weist ein feststehendes Außenrohr, ein Hohlrad (mit einer Innenverzahnung), eine exzentrische Sicherungsbuchse und ein Ritzel (mit einer Außenverzahnung, die mit der Innenverzahnung des Hohlrads kämmt), das mit dem Hohlrad in Kämmeingriff steht, auf. Der relativ abwinkelbare distale Schaftabschnitt weist weiter bevorzugt ein flexibles Übertragungselement, eine Verstellbuchse mit einem Mitnahmezapfen und eine distale Schaftspitze auf. Der distale Schaftabschnitt ist vorzugsweise mit dem Lager auf dem proximalen Abschnitt gelagert. Der flexible Fräser ist vorzugsweise durch den proximalen Schaftabschnitt und den distalen Schaftabschnitt geführt und ist zusammen mit dem distalen Schaftabschnitt abwinkelbar. Die Verstellbuchse ist vorzugsweise durch das flexible Übertragungselement mit dem Ritzel derart verbunden, dass eine Rotationsbewegung des Ritzels auf die Verstellbuchse übertragen wird. Die Verstellbuchse überträgt vorzugsweise die Drehung durch den Mitnahmezapfen auf die distale Schaftspitze. Durch die Drehung des distalen Schaftabschnitts relativ zum angestellten proximalen Schaftabschnitt wird der distale Schaftabschnitt abgewinkelt.

Gemäß einem weiteren bevorzugten Merkmal der Offenbarung weist der proximale Schaftabschnitt das Hohlrad mit der Innenverzahnung auf, die mit der Außenverzahnung des Ritzels kämmt. Der proximale Schaftabschnitt weist bevorzugt das feststehende Außenrohr mit der angestellten Stirnseite auf. In dem feststehenden Außenrohr ist das Hohlrad um seine Längsachse drehbar gelagert. Das Hohlrad ist bevorzugt vom proximalen Endabschnitt des Schaftes aus bedienbar und weist die Innenverzahnung auf. Die Innenverzahnung kämmt mit der Außenverzahnung des Ritzels. In anderen Worten ausgedrückt ist die Innenverzahnung in operativem Eingriff mit der Außenverzahnung. Dadurch wird eine Rotation des Hohlrads auf das Ritzel übertragen.

Gemäß einem weiteren bevorzugten Merkmal der Offenbarung hat die Verstellbuchse den Mitnahmezapfen, der formschlüssig mit der distalen Schaftspitze verbunden ist und die Rotation der Verstellbuchse auf die distale Schaftspitze überträgt. Die Verstellbuchse ist bevorzugt mit der distalen Schaftspitze durch den Mitnahmezapfen verbunden. Die Rotation der Verstellbuchse wird dadurch auf die distale Schaftspitze übertragen und die distale Schaftspitze gedreht. Im Endeffekt wird die distale Schaftspitze also bevorzugt mit dem Hohlrad mitgedreht. Durch die Drehung der distalen Schaftspitze stellen sich die angestellten Stirnseiten des distalen Schaftabschnitts und des proximalen Schaftabschnitts derart gegeneinander, dass der distale Schaftabschnitt abgewinkelt wird.

Gemäß einem weiteren bevorzugten Merkmal der Offenbarung weist der proximale Schaftabschnitt eine exzentrische Sicherungsbuchse auf. Die exzentrische Sicherungsbuchse ist vorzugsweise in dem Außenrohr angebracht. Durch die exzentrische Sicherungsbuchse wird das Ritzel an eine Seite des Außenrohrs gedrückt, die der exzentrischen Sicherungsbuchse gegenüberliegt. Das Ritzel ist bevorzugt an der Seite des Schafts, in die der distale Schaftabschnitt nicht abgewinkelt wird. Das Ritzel wird durch das Hohlrad angetrieben und dreht sich in der exzentrischen Sicherungsbuchse. Dadurch ist das Ritzel immer an der Seite des Außenrohrs angeordnet. Da das Ritzel bevorzugt an der Seite angeordnet ist, in die sich der distale Schaftabschnitt nicht biegt, ist das flexible Übertragungselement weiter vom flexiblen Fräser entfernt.

Gemäß einem weiteren bevorzugten Merkmal der Offenbarung ist das flexible Übertragungselement ein flexibles Federblech vorzugsweise mit einer seitlich angebrachten Kugel. Das flexible Übertragungselement kann als ein eierndes/auf einer Kreisbahn umlaufendes Blech ausgeführt sein. Wenn das Federblech mit dem Ritzel rotiert, rotiert es nicht um seine eigene Längsachse, sondern eiert um eine Längsachse des Ritzels. Dadurch ist das Federblech in der um 45° abgewinkelten Stellung am weitesten vom flexiblen Fräser entfernt. Dadurch ist das Risiko einer Kollision minimiert und der Aufbau des Schafts kann kleiner ausgeführt werden.

Gemäß einem weiteren bevorzugten Merkmal der Offenbarung ist die Kugel des flexiblen Federblechs in einer kugelförmigen Aufnahmenut in dem Ritzel aufgenommen und die der Kugel gegenüberliegende Seite des Federblechs ist bevorzugt mit der Verstellbuchse verbunden.

Gemäß einem weiteren bevorzugten Merkmal der Offenbarung ist die Kugel des flexiblen Federblechs in einer kugelförmigen Aufnahmenut in dem Ritzel aufgenommen ist und die der Kugel gegenüberliegende Seite des flexiblen Federblechs ist vorzugsweise mit dem Ritzel verbunden.

Die Kugel ist bevorzugt bewegbar in der Aufnahmenut aufgenommen. Die Kugel ist bevorzugt formschlüssig in der Aufnahmenut befestigt. Dabei kann die Kugel sich aber in eine Längsrichtung der Aufnahmenut bewegen. Die Aufnahmenut kann in dem Ritzel oder in der Verstellbuchse angebracht sein. Auf der Seite des Federblechs, die der Kugel gegenüberliegt ist das Federblech mit dem entsprechenden Bauteil vorzugsweise verschweißt oder verklebt.

Gemäß einem weiteren bevorzugten Merkmal der Offenbarung ist das flexible Übertragungselement ein Silikonschlauch. Vorzugsweise ist der Silikonschlauch ebenfalls mit der Verstellbuchse und dem Ritzel verbunden. Der Silikonschlauch wird vorzugsweise durch Kleben an der Verstellbuchse und dem Ritzel befestigt.

Gemäß einem weiteren bevorzugten Merkmal der Offenbarung ist das flexible Übertragungselement ein flexibler Metallfaltenbalg. Der flexible Metallfaltenbalg hat Falten, die den Falten einer Ziehharmonika oder eines Blasebalgs ähneln. Dadurch ist der Metallfaltenbalg flexibel und dehnbar.

Gemäß einem weiteren bevorzugten Merkmal der Offenbarung ist das flexible Übertragungselement ein flexibles Metallrohr. Das Metallrohr hat vorzugsweise Aussparungen bzw. eine Spaltgeometrie, die das Metallrohr flexibel machen.

Beide Lösungen mit Metallrohren als Basis haben folgende Vorteile:
- Die Metallrohre sind sehr torsionssteif bei gleichzeitiger guter Biegbarkeit. Dadurch ist eine sehr präzise Verstellung bzw. stabile Position des distalen Schaftabschnitts möglich.
- Bei der Spaltgeometrie wird die Torsionsteifigkeit durch die spezielle Anordnung der Spalte erreicht.
- In Drehrichtung gibt es keine Unterbrechung der Kontur und dadurch kein Umkehrspiel.
- Beide Lösungen mit Metallrohren als Basis können mit der Verstellbuchse bzw. dem Ritzel verschweißt oder geklebt werden.

Gemäß einem weiteren, bevorzugten Merkmal der Offenbarung ist ein Abknickwinkel zwischen dem proximalen Schaftabschnitt und dem distalen Schaftabschnitt doppelt so groß, wie der Anstellwinkel der angestellten Stirnseiten. Im abgewinkelten Zustand liegen die angestellten Stirnseiten derart aneinander an, dass sich die Anstellwinkel addieren. Da beide Stirnseiten den gleichen Anstellwinkel haben, ist der Abknickwinkel doppelt so groß, wie der Anstellwinkel. Durch die Verdopplung ergeben sich große Abknickwinkel, ohne große Anstellwinkel zu fordern.

Gemäß einem weiteren bevorzugten Merkmal der Offenbarung hat der Abknickwinkel zwischen dem proximalen Schaftabschnitt und dem distalen Schaftabschnitt ein Maximum und der Anstellwinkel wird bei einer weiteren Rotation des distalen Schaftabschnitts wieder kleiner. Wenn der distale Schaftabschnitt weiter rotiert wird, stehen die angestellten Stirnseiten nicht mehr direkt aufeinander. Deshalb nimmt der Abknickwinkel bei einer weiteren Rotation wieder ab.

Gemäß einem weiteren bevorzugten Merkmal der Offenbarung ist die Verstellbuchse aus einem Gleitlagerwerkstoff, vorzugsweise PTFE oder POM, gefertigt und/oder weist eine Beschichtung mit PTFE auf. Die Verstellbuchse rotiert bevorzugt in einer Aufnahmebohrung des Außenrohrs. Vorzugsweise ist in der Aufnahmebohrung kein weiteres Lager angeordnet. Deshalb muss die Verstellbuchse gleiten. Durch die Fertigung aus dem Gleitlagerwerkstoff hat die Verstellbuchse eine kleinere Reibung bezüglich der Aufnahmebohrung.

Gemäß einem weiteren bevorzugten Merkmal der Offenbarung ist die distale Schaftspitze aus einem Kunststoff mit guten Gleiteigenschaften, vorzugsweise PTFE oder POM, gefertigt. Wenn zwischen dem Außenrohr und der distalen Schaftspitze kein Lager angebracht ist, muss die distale Schaftspitze bezüglich dem Außenrohr drehbar sein. Das wird durch Materialauswahl der distalen Schaftspitze sichergestellt.

Gemäß einem weiteren bevorzugten Merkmal der Offenbarung ist die distale Schaftspitze aus einem flexiblen Kunststoff gefertigt. Die distale Schaftspitze ist derart gefertigt, dass sie biegbar ist. Damit kann die distale Schaftspitze mit dem Außenrohr eine Hinterrastung/Hinterschneidung bilden, die die distale Schaftspitze an dem proximalen Schaftabschnitt fixiert. Für die Montage wird die distale Schaftspitze aufgebogen und mit dem Außenrohr verrastet.

Gemäß einem weiteren bevorzugten Merkmal der Offenbarung wird über das Einstellrad manuell oder motorisch eine gewünschte Winkelstellung eingestellt. Das Einstellrad befindet sich vorzugsweise am proximalen Ende des Handinstruments. Damit kann ein Benutzer einen Wunschwinkel einstellen. Der Benutzer kann beispielsweise an einem Rädchen drehen, das das Einstellelement darstellt, oder der Benutzer kann den Wunschwinkel an einem Hebel, Joystick oder Ähnliches einstellen.

Gemäß einem weiteren bevorzugten Merkmal der Offenbarung ist das Lager ein Vollkugellager. Durch ein Vollkugellager wird die Reibung in dem Lager verringert. Dadurch gibt es weniger Verluste beim Einstellen des Winkels. Ebenso kann dadurch das notwendige Spiel für eine leichtgängige Verstellbarkeit minimiert werden und somit die Präzision der distalen Spitze erhöht werden.

Gemäß einem weiteren bevorzugten Merkmal der Offenbarung ist das Wälzlager ein Kugellager mit mindestens, vorzugsweise genau drei Kugeln. Durch die Verwendung von drei oder auch mehreren Kugeln erhöht sich die Reibung im Wälzlager. Vorteilhaft ist aber, dass die Montage des Wälzlagers schneller geht, da weniger Kugeln eingefüllt werden müssen und, dass das Wälzlager kostengünstiger ist.

### Kurzbeschreibung der Figuren

Fig. 1 zeigt einen Schaft gemäß einer ersten Ausführungsform in einer geraden Schaftform;
Fig. 2 zeigt den Schaft gemäß der ersten Ausführungsform bei dem ein distaler Schaftabschnitt im Vergleich zu einem proximalen Schaftabschnitt um 22,5° abgewinkelt ist;
Fig. 3 zeigt den Schaft gemäß der ersten Ausführungsform bei dem der distale Schaftabschnitt im Vergleich zu dem proximalen Schaftabschnitt um 45° abgewinkelt ist;
Fig. 4 zeigt einen Längsschnitt durch den geraden Schaft;
Fig. 5 zeigt einen Längsschnitt durch den um 22,5° abgewinkelten Schaft;
Fig. 6 zeigt einen Längsschnitt durch den um 45° abgewinkelten Schaft;
Fig. 7 zeigt einen Querschnitt durch den proximalen Schaftabschnitt;
Fig. 8 zeigt einen Querschnitt durch ein Wälzlager, das zwischen dem proximalen Schaftabschnitt und dem distalen Schaftabschnitt angeordnet ist;
Fig. 9 zeigt eine isometrische Ansicht eines Federblechs mit einer Kugel;
Fig. 10 zeigt eine Seitenansicht des Federblechs;
Fig. 11 zeigt das Federblech in dem geraden Schaft;
Fig. 12 zeigt das Federblech in dem um 45° abgewinkelten Schaft;
Fig. 13 zeigt eine Draufsicht auf das Federblech in dem um 22,5° abgewinkelten Schaft;
Fig. 14 zeigt das tordierte Federblech in dem um 22,5° abgewinkelten Schaft;
Fig. 15 zeigt einen Querschnitt durch den proximalen Schaftabschnitt;
Fig. 16 zeigt eine isometrische Ansicht eines flexiblen Silikonschlauchs gemäß der ersten Ausführungsform;
Fig. 17 zeigt eine Seitenansicht des flexiblen Silikonschlauchs gemäß der ersten Ausführungsform;
Fig. 18 zeigt eine isometrische Ansicht eines flexiblen Metallfaltenbalgs gemäß einer dritten Ausführungsform;
Fig. 19 zeigt eine Seitenansicht des flexiblen Metallfaltenbalgs gemäß der dritten Ausführungsform;
Fig. 20 zeigt eine isometrische Ansicht eines flexiblen Metallrohrs gemäß einer vierten Ausführungsform;
Fig. 21 zeigt eine Seitenansicht des flexiblen Metallrohrs gemäß der vierten Ausführungsform;
Fig. 22 zeigt einen Längsschnitt durch den geraden Schaft mit einem Wälzlager gemäß einer fünften Ausführungsform;
Fig. 23 zeigt einen Querschnitt durch das Wälzlager gemäß der fünften Ausführungsform;
Fig. 24 zeigt einen Längsschnitt durch einen geraden Schaft gemäß einer sechsten Ausführungsform;
Fig. 25 zeigt einen Querschnitt durch einen distalen Schaftabschnitt gemäß der sechsten Ausführungsform;
Fig. 26 zeigt einen Schaft gemäß einer siebten Ausführungsform; und
Fig. 27 zeigt ein Handinstrument mit dem offenbarungsgemäßen abwinkelbaren Schaft.

### Beschreibung der Ausführungsformen

Nachstehend werden bevorzugte Ausführungsformen der vorliegenden Offenbarung auf der Basis der zugehörigen Figuren beschrieben.

### Erste Ausführungsform

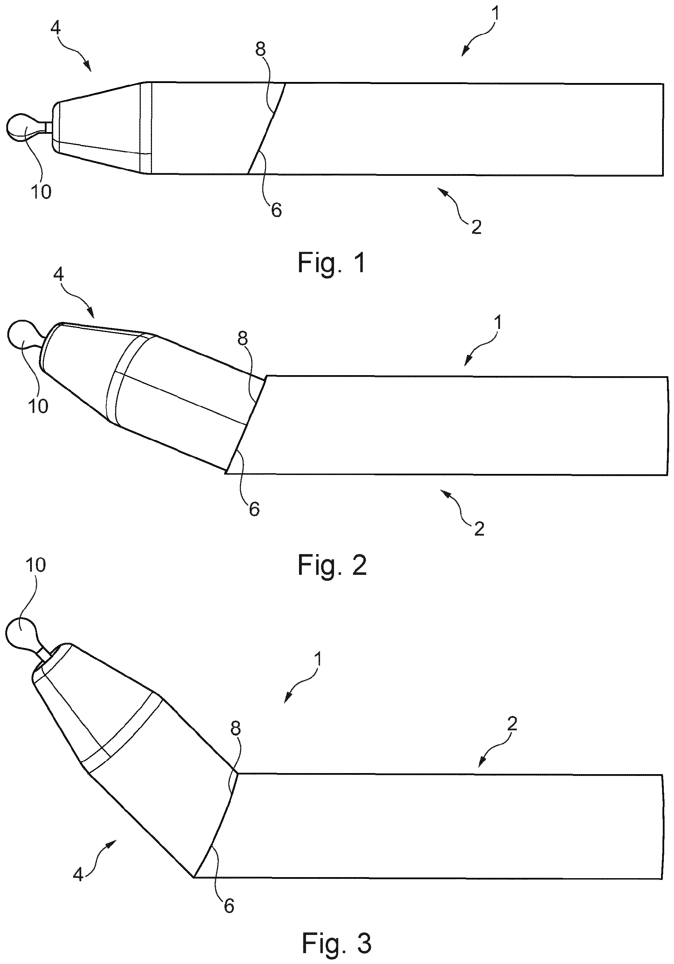

Fig. 1 zeigt einen Schaft 1 eines oder für ein medizinisches Handinstrument in einer geraden Schaftform. D.h. ein proximaler Schaftabschnitt 2 des Schafts 1 und ein distaler Schaftabschnitt 4 des Schafts 1 sind in einer Linie angeordnet bzw. weisen einen Winkel von 0° zueinander auf. Der proximale Schaftabschnitt 2 ist dabei im Wesentlichen rohrförmig und weißt an seinem distalen Endabschnitt eine in Schaftlängsachse angestellte Stirnseite 6 auf. Der distale Schaftabschnitt 4 ist ebenfalls annähernd rohrförmig. Das Rohr läuft zum distalen Ende zu einer Spitze zusammen. Der distale Schaftabschnitt 4 weist an seinem proximalen Endabschnitt eine in Schaftlängsachse angestellte Stirnseite 8 auf. Ein flexibler Fräser 10 ragt am distalen Ende aus dem Schaft 1. Der flexible Fräser 10 verläuft entlang der Schaftlängsachse. Es ist offensichtlich, dass statt dem flexiblen Fräser 10 ein Bohrer oder ein sonstiges medizinisches Werkzeug aus dem Schaft 1 ragen kann.

Die angestellten Stirnseiten 6, 8 weisen jeweils einen Anstellwinkel von vorzugsweise 22,5° zu einer Normalenebene zur Schaftlängsachse auf. Wenn der Schaft 1 in einer geraden Schaftform bzw. ausgestreckt ist, sind die beiden angestellten Stirnseiten 6, 8 derart zueinander versetzt, dass sich die langen Enden der angestellten Stirnseiten 6, 8 in Relation zur Längsachse gegenüberliegen. Die angestellten Stirnseiten liegen aufeinander auf. Die angestellten Stirnseiten müssen nicht zwangsläufig einen Anstellwinkel von 22,5° aufweisen. Es sind auch Anstellwinkel von beispielsweise 10°, 18°, 30°, 45° oder jeder andere Anstellwinkel möglich.

Fig. 2 zeigt den Schaft 1, wobei der distale Schaftabschnitt 4 zu dem proximalen Schaftabschnitt 2 um 22,5° abgewinkelt ist. Der distale Schaftabschnitt 4 ist im Vergleich zur Stellung in Fig. 1 um 90° um die eigene Längsachse relativ zum proximalen Schaftabschnitt 2 gedreht. Dabei liegen die angestellten Stirnseiten 6,8 nicht vollständig / flächig aufeinander auf. Die angestellte Stirnseite 8 ist mit dem distalen Schaftabschnitt 4 um 90° relativ zur angestellten Stirnseite 6 des proximalen Schaftabschnitts 2 gedreht, somit steht ein langes Ende der angestellten Stirnseite 8 über die angestellte Stirnseite 6 hinaus. Durch den Anstellwinkel der angestellten Stirnseite 6 steht der distale Schaftabschnitt 4 relativ zum proximalen Schaftabschnitt 2 nach oben. Der flexible Fräser 10 wird mit dem distalen Schaftabschnitt 4 zusammen abgewinkelt.

Fig. 3 zeigt den Schaft 1, wobei der distale Schaftabschnitt 4 zu dem proximalen Schaftabschnitt 2 bei einem jeweiligen Anstellwinkel der beiden Stirnseiten um 22,5° folglich um 45° abgewinkelt ist. Der distale Schaftabschnitt 4 ist im Vergleich zur Stellung in Fig. 1 um 180° um die eigene Längsachse gedreht. Die angestellten Stirnseiten 6, 8 liegen in dieser Stellung wieder komplett / flächig aufeinander auf. Durch die Drehung des distalen Schaftabschnitts 4 sind die langen Enden der angestellten Stirnseiten 6, 8 aber nebeneinander positioniert. Die Anstellwinkel der angestellten Stirnseiten 6, 8 addieren sich somit. Dadurch wird der distale Schaftabschnitt 4 im Vergleich zum proximalen Schaftabschnitt 2 um den doppelten Anstellwinkel der angestellten Stirnseiten 6, 8 abgewinkelt.

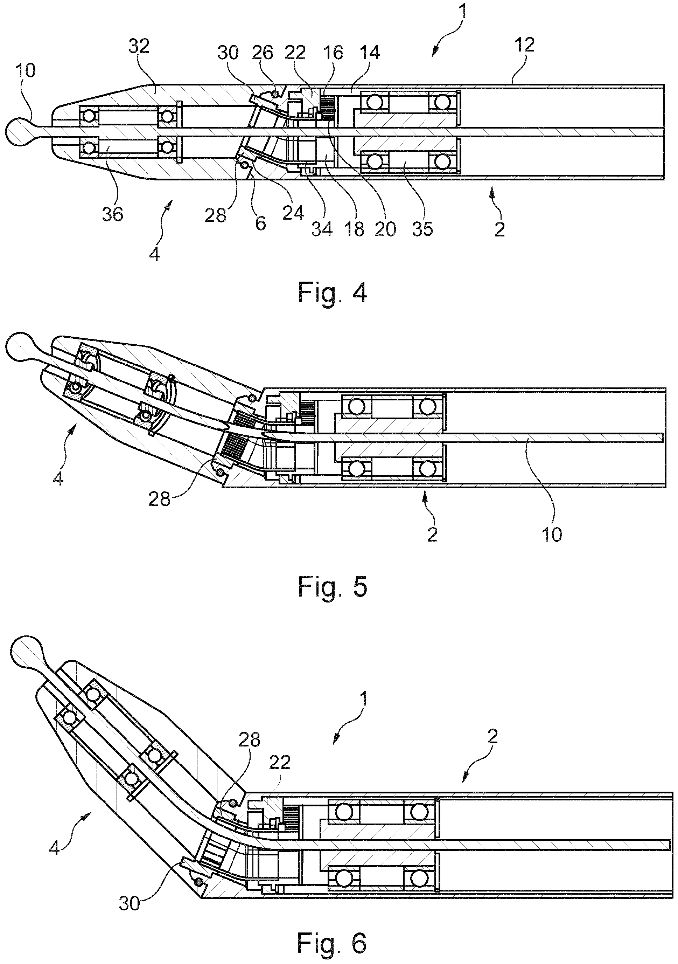

Fig. 4 zeigt einen Querschnitt des ausgestreckten (geraden) Schafts 1 durch eine Längsachse. Der proximale Schaftabschnitt 2 weist ein feststehendes Außenrohr 12, ein Hohlrad 14 mit einer Innenverzahnung 16, ein Ritzel 18 mit einer Außenverzahnung 20 und eine exzentrische Sicherungsbuchse 22 auf. Das Hohlrad 14 ist innerhalb des Außenrohrs 12 positioniert und die Längsachse des Außenrohrs 12 entspricht der Längsachse des Hohlrads 14. Das Außenrohr 12 und das Hohlrad 14 sind also konzentrisch angeordnet. Das Hohlrad 14 ist mit einem proximalen Einstellrad (nicht dargestellt) verbunden und dreht sich mit dem Einstellrad mit. An dem Einstellrad kann ein Nutzer eine gewünschte Winkelstellung einstellen. Die Winkelstellung kann entweder manuell oder durch einen Motor unterstützt eingestellt werden. Ebenso kann das proximale Einstellrad eine Arretierung (nicht dargestellt) (beispielsweise ein Kugeldruckstück, eine Klemmschraube oder ein Rastring) aufweisen, welche die Verstellung des distalen Schaftabschnitts 4 blockiert. So wird sichergestellt, dass die Verstellung nur durch aktives, beabsichtigtes Handeln erfolgt. Das Außenrohr 12 bewegt sich nicht. Das distale Ende des Außenrohrs 12 weist die angestellte Stirnseite 6 auf. Das distale Ende des Außenrohrs 12 weist weiterhin eine Aufnahmebohrung 24 und einen Aufnahmezapfen für ein Wälzlager 26 auf. Das Außenrohr weist eine Rille/ Nut für die Kugeln des Wälzlagers 26 auf. Auf dem Wälzlager 26 ist der distale Schaftabschnitt 4 gelagert.

Die Innenverzahnung 16 kämmt mit der Außenverzahnung 20 des Ritzels 18. Dadurch wird eine Rotation des Hohlrads 14, die durch das Einstellrad gesteuert wird, auf das Ritzel 18 übertragen. Die Drehrichtung des Ritzels 18 ist dabei entgegengesetzt der Drehrichtung des Hohlrads 14. Das Ritzel 18 wird vom Hohlrad 14 angetrieben, das Ritzel dreht sich aber in der exzentrischen Sicherungsbuchse 22. Die Sicherungsbuchse 22 ist exzentrisch zum Hohlrad 14 angeordnet. D.h. die Längsachse der exzentrischen Sicherungsbuchse 22 ist zwar parallel zur Längsachse des Hohlrads 14, die Längsachsen liegen aber nicht übereinander.

Der distale Schaftabschnitt 4 weist eine Verstellbuchse 28 mit einem Mitnahmezapfen 30 und eine distale Schaftspitze 32 auf. Die distale Schaftspitze 32 ist auf dem Wälzlager 26 gelagert. Die Verstellbuchse 28 ist in der Aufnahmebohrung 24 des proximalen Schaftabschnitts 2 gelagert. Der Mitnahmezapfen 30 der Verstellbuchse 28 greift formschlüssig in die distale Schaftspitze 32 ein. Die Verstellbuchse 28 ist über einen flexiblen Silikonschlauch 34 mit dem Ritzel 18 derart verbunden, dass eine Rotation des Ritzels 18 auf die Verstellbuchse 28 übertragen wird. Der flexible Silikonschlauch 34 ist dabei ein anspruchsgemäßes flexibles Übertragungselement. Der flexible Silikonschlauch 34 ist an der Verstellbuchse 28 und dem Ritzel 18 beispielsweise durch Schweißen oder Kleben befestigt. Da die Verstellbuchse 28 über den Mitnahmezapfen 30 mit der distalen Schaftspitze 32 formschlüssig verbunden ist, wird eine Rotation der Verstellbuchse 28 auf die distale Schaftspitze 32 übertragen.

Der flexible Fräser 10 verläuft sowohl durch den proximalen Schaftabschnitt 2 als auch durch den distalen Schaftabschnitt 4. Der flexible Fräser 10 ist jeweils in den proximalen Schaftabschnitt 2 und in den distalen Schaftabschnitt 4 durch Wälzlager 35, 36 gelagert. Der flexible Fräser 10 ist mit dem distalen Schaftabschnitt 4 abwinkelbar.

Über diese Anordnung kann eine stufenlose Verstellung des distalen Schaftabschnitts 4 zwischen 0 und 45 Grad realisiert werden. Das vollkugelige Wälzlager 26 stellt sicher, dass auch bei belasteter Instrumentenspitze eine leichtgängige und ruckfreie (kein Stick-/ Slip-Effekt) Verstellung möglich ist.

Um den Schaft 1 zu montieren, wird zuerst die Sicherungsbuchse 16 in das Außenrohr 12 eingebracht. Anschließend wird das Hohlrad 14 mit dem Ritzel 18 in das Außenrohr gesteckt. Danach wird das Lager 34 für den flexiblen Fräser 10 montiert und mit einem Sicherungsring befestigt. In die Aufnahmebohrung 24 wird die Verstellbuchse 28 mit dem flexiblen Übertragungselement 34 eingesetzt. Das Wälzlager 26 wird auf das Außenrohr 12 aufgesetzt. Die distale Schaftspitze 32 wird auf das Wälzlager 26 aufgesetzt. Der Mitnahmezapfen 30 der Verstellbuchse 28 greift formschlüssig in die distale Schaftspitze 32 ein.

Fig. 5 zeigt einen Querschnitt durch den um 22,5° angewinkelten Schaft 1. Dabei ist der distale Schaftabschnitt 4 im Vergleich zu dem proximalen Schaftabschnitt 2 um 22,5° abgewinkelt. Es ist anzumerken, dass der Mitnahmezapfen 30 in dieser Querschnittsansicht nicht zu sehen ist, da sich der Mitnahmezapfen 30 mit der Verstellbuchse 28 dreht und in dieser Ansicht von der Verstellbuchse 28 verdeckt wird. Der flexible Fräser 10 wird mit dem proximalen Schaftabschnitt 2 verdreht. Der distale Schaftabschnitt 4 bewegt sich auf einer Kreisbahn in die 22,5 Grad Stellung. Das Hohlrad 14 und das Ritzel 18 drehen sich jeweils in die entgegengesetzte Richtung.

Eine geringe Reibung der Verstellbuchse 28 lagernden Aufnahmebohrung 24 ist vorteilhaft. Dies lässt sich durch einen Gleitlagerwerkstoff (beispielsweise PTFE, POM) oder durch eine Beschichtung (beispielsweise PTFE) der Verstellbuchse 28 erreichen.

Fig. 6 zeigt einen Querschnitt durch den angewinkelten Schaft 1 mit einer Winkelung von 45°. Der Mitnahmezapfen 30 ist in dieser Stellung gegenüber der Sicherungsbuchse 22 angeordnet. Das heißt, die Verstellbuchse 28 mit dem Mitnahmezapfen 30 hat sich von der ausgestreckten Position zur maximalen Abwinklung um 180° gedreht.

Die 45°-Position stellt für diese Konstruktion den Umkehrpunkt dar. Die Verstellbuchse 28 hat sich in dieser Position um 180° gedreht. Bei weiterer Drehung des Hohlrads 14 dreht sich der distale Schaftabschnitt 4 wieder in die Ausgangsstellung (0°-Position) zurück. Je nach Anwendung kann dies vorteilhaft oder auch unnötig sein. Im zweiten Fall wäre eine Drehrichtungsumkehr zur Rückkehr in die Ausgangslage die Folge. Da sich das Instrument im Arbeitskanal des Endoskops um 360 Grad um seine eigene Achse drehen lässt, kann dennoch jede Position erreicht werden.

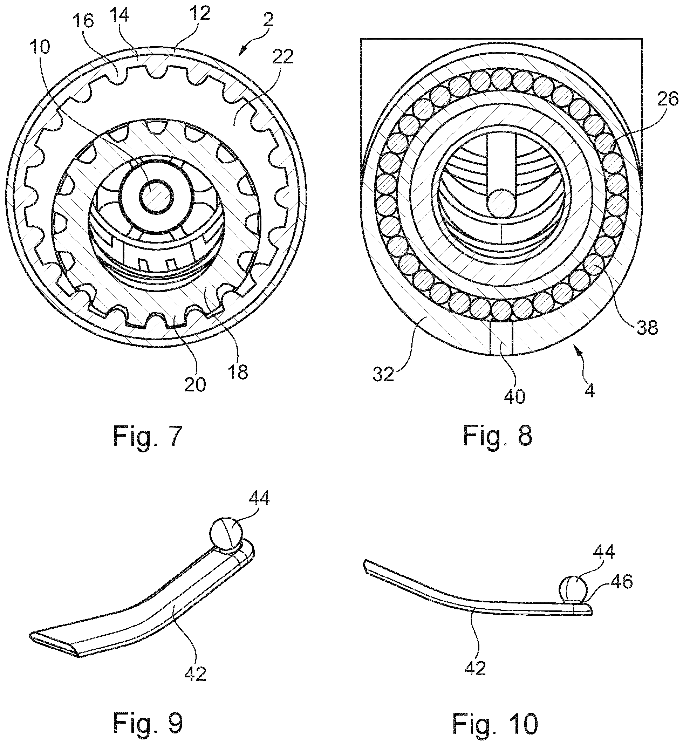

Fig. 7 zeigt einen Querschnitt durch den proximalen Schaftabschnitt 2. Fig. 7 zeigt das Hohlrad 14 mit der Innenverzahnung 16 in dem Außenrohr 12. Das Ritzel 18 mit der Außenverzahnung 20 kämmt mit der Innenverzahnung 16. Die exzentrische Sicherungsbuchse 22 ist an der Oberseite des Außenrohrs 12 angebracht und ist in Kontakt mit dem Ritzel 18. Dadurch verbleibt das Ritzel 18 auch bei der Rotation des Hohlrads 14 an der Unterseite des Außenrohrs 12. Die Unterseite des Außenrohrs 12 ist dabei die Seite, die der exzentrischen Sicherungsbuchse 22 gegenüberliegt. D.h. die exzentrische Sicherungsbuchse 22 drückt das Ritzel 18 zur Unterseite des Außenrohrs 12.

Fig. 8 zeigt einen Querschnitt durch den distalen Schaftabschnitt 4 und das Wälzlager 26. Bei dem Wälzlager 26 handelt es sich bevorzugt um ein Vollkugellager. Durch die Gestaltung des Wälzlagers 26 als ein Vollkugellager ist für eine minimale Friktion im Wälzlager 26 gesorgt. Für die Montage des Wälzlagers 26 werden Kugeln 38 durch die Befüllöffnung 40 in die Rillenbahn eingelassen. Ein rechnerischer Freiraum von 1 bis 2 Kugeln ist vorteilhaft für ein leichtgängiges Abrollen der Wälzkörper. Die Befüllöffnung 40 kann mit einem Zylinderstift verschlossen und der Zylinderstift unlösbar verschweißt sein.

### Zweite Ausführungsform

In einer zweiten Ausführungsform wird das flexible Übertragungselement durch ein Federblech 42 mit einer seitlich angebrachten Kugel 44 realisiert. Fig. 9 zeigt das Federblech 42 in einer isometrischen Ansicht. Das Federblech 42 ist flexibel ausgeführt und hat einen seitlichen Stift 46, auf dem die Kugel 44 angebracht ist. Der Stift 46 ist entweder fest mit dem Federblech 42 verschweißt oder der Stift 46 ist um seine eigene Längsachse drehbar mit dem Federblech 42 verbunden.

Im bevorzugten Ausführungsbeispiel ist ein flexibles Übertragungselement dargestellt. Dieses kann in unterschiedlicher Form realisiert werden. Bevorzugte Variante ist das Federblech 42 mit der seitlich angebrachten Kugel 44. Dieses ist besonders positionsstabil und besitzt eine hohe Wiederholgenauigkeit. Weiterhin benötigt das Federblech 42 durch seine seitliche Anbringung nur wenig Platz und bietet folglich mehr Platz für die Durchführung des flexiblen Fräsers 10. Das flexible Übertragungselement ist das flexible Federblech 42 mit dem seitlich angebrachten Stift 46 mit der Kugelkuppe. Fig. 10 zeigt eine Seitenansicht des Federblechs 42. Das Federblech 42 ist flexibel. Dabei ist das Federblech 42 sowohl um eine Achse, die senkrecht zur Längsachse des Federblechs 42 verläuft, biegbar als auch um die Längsachse des Federblechs 42.

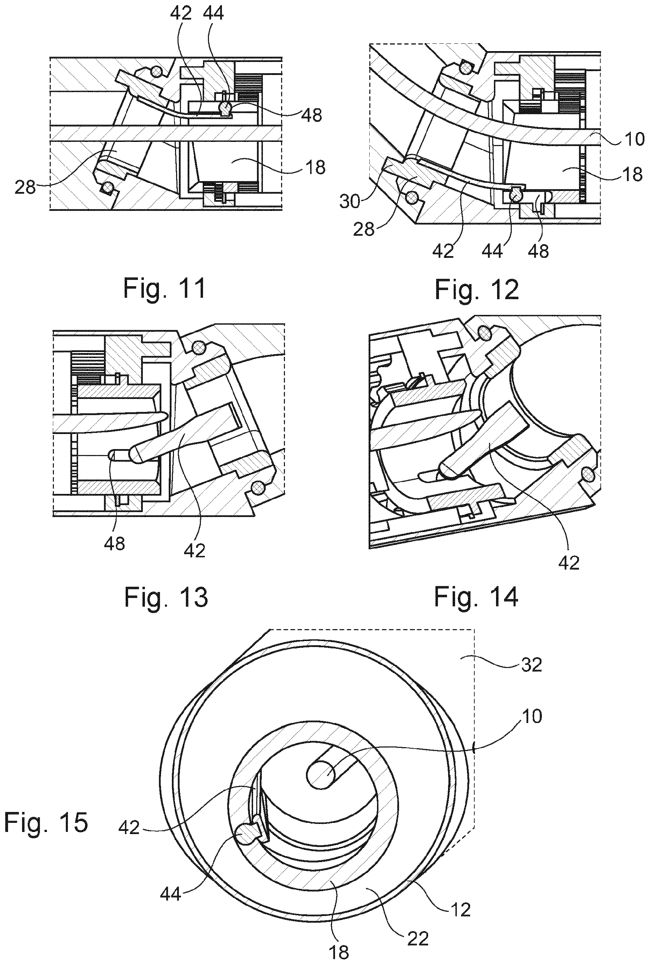

Fig. 11 zeigt das Federblech 42 in dem geraden Schaft 1. Dabei ist gezeigt, dass die Kugel 44 des Federblechs 42 in einer länglichen Aufnahmenut 48 in dem Ritzel 18 eingebracht ist. Die Aufnahmenut 48 verläuft in Längsrichtung des Schafts 1 und die Kugel 44 ist formschlüssig mit der Aufnahmenut 48 verbunden. Die Kugel 44 bewegt sich also mit der Rotation des Ritzels 18 mit und bewegt sich auch in Längsrichtung der Aufnahmenut 48. Die Seite des Federblechs 42, die der Kugel 44 gegenüberliegt, ist an der Verstellbuchse 28 befestigt. Eine Rotation des Ritzels 18 wird somit von dem Federblech 42 auf die Verstellbuchse 28 übertragen. Die Verstellbuchse 28 rotiert mit dem Federblech 42 mit. Das Federblech 42 ist beispielsweise durch Schweißen oder Kleben mit der Verstellbuchse 28 verbunden. Es ist anzumerken, dass die Aufnahmenut 48 auch in der Verstellbuchse 28 vorbereitet sein kann. In diesem Fall ist die Kugel 44 mit der Verstellbuchse 28 verbunden und das Federblech 42 mit dem Ritzel 18 verschweißt bzw. verklebt.

Die Kugel 44 bewegt sich in einer kugelförmigen Aufnahmenut 48 im Ritzel 18 in Längsrichtung der Aufnahmenut 48. Eine distale Fixierung in der Verstellbuchse 28 ist mittels Kleben oder Schweißen realisiert. Die proximale Fixierung entfällt. Durch den Formschluss der Kugel 44 in der Aufnahmenut 48 wird die Drehbewegung über das Federblech 42 auf die Verstellbuchse 28 übertragen.

Fig. 12 zeigt das Federblech 42 in dem um 45° abgewinkelten Schaft 1. Dabei ist das Ritzel 18 im Vergleich zum geraden Schaft 1 um 180° um seine Längsachse gedreht. Die Aufnahmenut 48 mit der Kugel 44 ist selbstverständlich auch um 180° mitgedreht. Die Verstellbuchse 28 ist durch das Federblech 42 auch mitgedreht. Der Mitnahmezapfen 30 nimmt die distale Schaftspitze 32 mit, die sich auch um 180° dreht. Dabei wird deutlich, dass das Federblech 42 im abgewinkelten Zustand mehr Abstand zu dem flexiblen Fräser 10 hat, als im ausgestreckten Zustand.

In den Figuren 11 und 12 ist die Position der Kugel 44 und die Biegung des Federblechs 42 in den beiden Endpositionen dargestellt. In der 45°-Position befindet sich das Federblech 42 unten. Gemeinsam mit einer exzentrischen Bohrung in der Verstellbuchse 28 und einer exzentrischen Fase am Ritzel 18 ist hier der Freiraum für den flexiblen Fräser 10 am größten. Dies ist der wesentliche Unterschied zu den nachfolgend aufgeführten Varianten und kann entscheidend bei der Miniaturisierung des Aufbaus sein.

Fig. 13 zeigt eine Draufsicht auf das Federblech 42. Der Schaft 1 ist dabei um 22,5° abgewinkelt. Durch die Rotation des Ritzels 18 wird das Federblech 42 mitgedreht und dreht die Verstellbuchse 28 mit. Dadurch wird das Federblech 42 verdreht. Es ist gezeigt, dass die Kugel 44 sich bei Rotation des Ritzels 18 in der Aufnahmenut 48 in Längsrichtung der Aufnahmenut 48 bewegt. Fig. 14 zeigt das Federblech 42. Dabei wird deutlich, dass das Federblech 42 bei der Rotation bzw. der Übertragung der Drehbewegung vom Ritzel 18 zur Verstellbuchse 28 um seine eigene Längsachse tordiert wird. In der 22,5°-Position wird ersichtlich, dass sich das Federblech 42 zur Aufnahmenut 48 verdreht und auch um seine Längsachse tordiert wird. Eine entsprechende Flexibilität des Federblechs 42 ist Grundvoraussetzung für die Funktion.

Fig. 15 zeigt einen Querschnitt durch den proximalen Schaftabschnitt 2 mit der exzentrischen Sicherungsbuchse 22 und dem Ritzel 18. Die Kugel 44 ist formschlüssig in der Aufnahmenut 48 eingebracht und folgt der Rotation der Aufnahmenut 48. Im Querschnitt erkennt man, dass die spezielle Aufnahmenut 48 mit Kugelform dafür sorgt, dass die Kugel 44 des Federblechs 42 über einen Formschluss gefasst ist und somit der Rotationsbewegung des Ritzels 18 folgen muss.

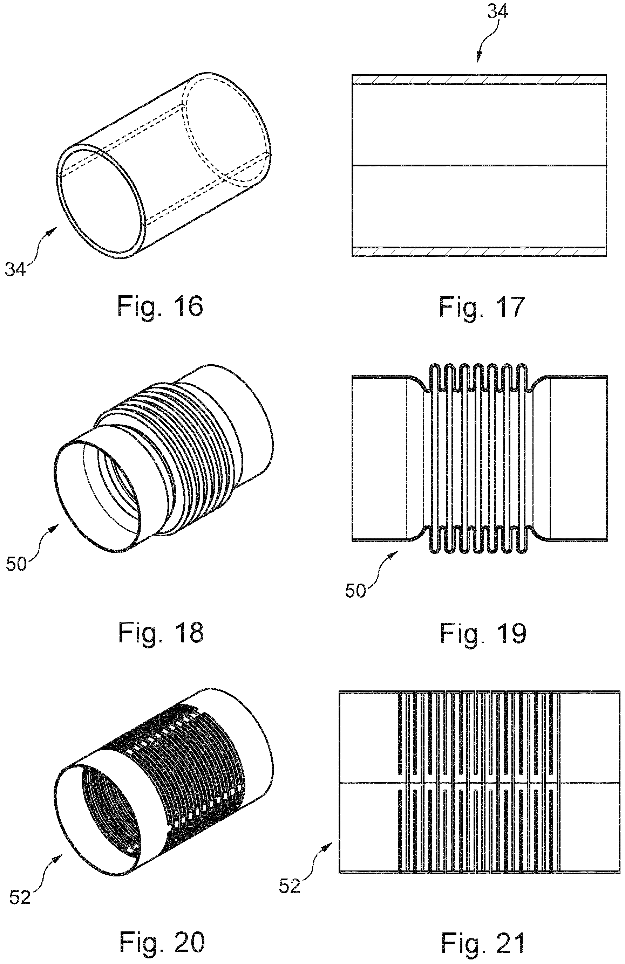

Fig. 16 zeigt den Silikonschlauch 34 gemäß der ersten Ausführungsform. Der Silikonschlauch 34 ist eine Möglichkeit das flexible Übertragungselement zu realisieren. Der Silikonschlauch 34 wird mit jeweils einem Ende an dem Ritzel 18 und der Verstellbuchse 28 verklebt. Fig. 17 zeigt eine Seitenansicht des Silikonschlauchs 34.

### Dritte Ausführungsform

Fig. 18 zeigt ein flexibles Übertragungselement gemäß einer dritten Ausführungsform. Das Übertragungselement ist ein flexibler Metallfaltenbalg 50. Der Metallfaltenbalg 50 ist im Wesentlichen ein hohles längliches Metallrohr. In der Mitte weist das Metallrohr Falten auf, die den Falten eines Blasebalgs oder einer Ziehharmonika ähneln. Durch die Falten ist der Metallfaltenbalg 50 biegbar bzw. flexibel. Der Metallfaltenbalg 50 kann an das Ritzel 18 und die Verstellbuchse 28 durch Kleben oder Schweißen befestigt werden. Fig. 19 zeigt eine Seitenansicht des Metallfaltenbalgs 50.

### Vierte Ausführungsform

Fig. 20 zeigt ein flexibles Übertragungselement gemäß einer vierten Ausführungsform. Das Übertragungselement ist ein flexibles Metallrohr 52. Das Metallrohr 52 ist im Wesentlichen ein dünnes längliches Metallrohr. Das Metallrohr 52 weist Schlitze/ Aussparungen auf, die in radialer Richtung des Metallrohrs 52 verlaufen. D.h. das Metallrohr 52 hat eine Spaltgeometrie. Die Aussparungen machen das Metallrohr 52 flexibel. Das Metallrohr 52 kann an das Ritzel 18 und die Verstellbuchse 28 durch Kleben oder Schweißen befestigt werden. Fig. 21 zeigt eine Seitenansicht des Metallrohrs 52.

### Fünfte Ausführungsform

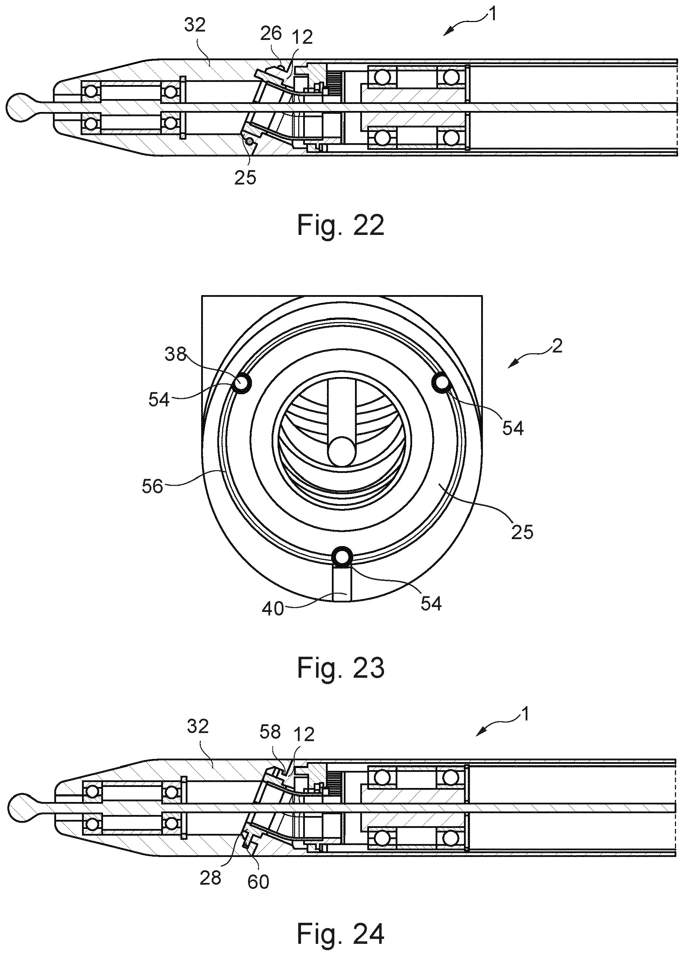

Fig. 22 zeigt einen ausgestreckten Schaft 1 gemäß einer fünften Ausführungsform. Gemäß der fünften Ausführungsform ist das Wälzlager 26 kein Vollkugellager, sondern weist lediglich drei oder auch mehr Kugeln 38 auf. Der Aufnahmezapfen 25 des Außenrohrs 12 weist drei Kugelbohrungen 54 auf. Die distale Schaftspitze 32 weist eine umlaufende Rille 56 auf. Die Kugeln 38 werden bei der Montage durch die Befüllöffnung 40 jeweils in die entsprechende Kugelbohrung 54 eingesetzt. Der Verschluss erfolgt analog zur ersten Ausführungsform. Die Kugeln 38 rotieren in ihrer jeweiligen Kugelbohrung 54. Dies führt zu einer leicht höheren Reibung, da die Abrollbewegung nur auf der Rille 56 der distalen Schaftspitze 32 möglich ist. Allerdings werden wesentlich weniger Kugeln 38 benötigt und die Montage ist schneller. Fig. 23 zeigt einen Querschnitt durch den distalen Schaftabschnitt 4 und das Wälzlager 26. Die Kugelbohrungen 54 sind in Umfangsrichtung gleich beabstandet.

### Sechste Ausführungsform

Fig. 24 zeigt einen ausgestreckten Schaft 1 gemäß einer sechsten Ausführungsform. Bei der sechsten Ausführungsform ist kein Wälzlager zwischen dem Außenrohr 12 und der distalen Schaftspitze 32 vorhanden. Vielmehr ist eine Gleitpaarung zwischen dem Außenrohr 12 und der distalen Schaftspitze 32 ausgebildet. Die distale Schaftspitze 32 ist durch eine Hinterrastung 58 mit dem Außenrohr 12 fixiert. Um die Hinterrastung 58 realisieren zu können, muss die distale Schaftspitze 32 aus einem flexiblen Material, wie beispielsweise Kunststoff, gefertigt sein. Die distale Schaftspitze 32 wird zur Montage aufgebogen und in die Vorsprünge des Außenrohrs 12 gerastet. Die Drehübertragung erfolgt durch einen seitlichen Mitnahmezapfen 60, da durch die fehlenden Kugeln Platz entstanden ist. Der Mitnahmezapfen 60 steht aus der Verstellbuchse 28 hervor und dreht sich mit der Verstellbuchse 28 mit. Das Außenrohr 12 weist eine Aussparung 61 auf, in der Mitnahmezapfen 60 bewegbar ist. Der Mitnahmezapfen 60 verläuft durch die Aussparung 61 und ist formschlüssig mit einer Ausbuchtung der distalen Schaftspitze 32 verbunden.

Bei Verwendung eines Kunststoffs mit guten Gleiteigenschaften (beispielsweise PTFE, POM) kann auch ohne Wälzlager eine leichtgängige Verstellung realisiert werden. Diese Variante eignet sich besonders für kostengünstige Single Use Instrumente.

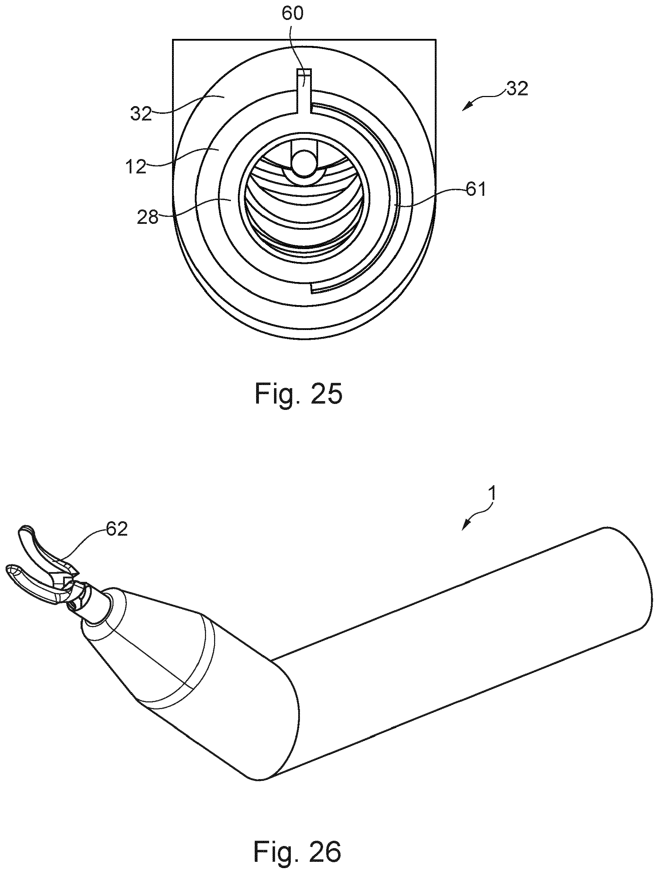

Fig. 25 zeigt einen Querschnitt durch die distale Schaftspitze 32. Die Verstellbuchse 28 weist den hervorstehenden Mitnahmezapfen 60 auf, der in die distale Schaftspitze 32 eingreift. Der Mitnahmezapfen 60 durchstößt das Außenrohr 12 durch die Aussparung 61. Die Aussparung 61 umfasst den halben Umfang des Außenrohrs 12. Der Mitnahmezapfen 60 überträgt die Rotation der Verstellbuchse 28 auf die distale Schaftspitze 32.

### Siebte Ausführungsform

Fig. 26 zeigt einen abgewinkelten Schaft 1 gemäß einer siebten Ausführungsform. Dabei weist der Schaft 1 keinen Fräser auf, sondern hat Maulteile oder Scherenblätter 62, die aus dem distalen Schaftabschnitt ragen. Mit den Maulteilen 62 kann ein Benutzer Objekte greifen oder fixieren.

Bei endoskopischen Instrumenten mit winkelbarer Spitze spielt die Reibung der flexiblen Ansteuerung eine untergeordnete Rolle, da in der Regel keine hohen Frequenzen für die Bewegung der Maulteile oder Scherenblätter 62 erreicht werden (< 100 Betätigungen pro Minute).

Hier können dann auch größere Abwinkelungen erreicht werden, ohne dass eine unzulässige Erwärmung des Schaftes eintritt. Durch einen Schrägschnitt von 45 Grad (anstatt von 22,5 Grad) ist eine maximale Abwinkelung von 90 Grad realisierbar (nicht dargestellt). Allerdings erhöht sich durch die stärkere elliptische Ausprägung der Schnittfläche der seitliche Überstand der Schaftkanten im Bereich des Drehgelenkes (besonders in der Zwischenposition auf der Hälfte des Verstellbereiches). Durch spezielle Rundungen kann der Überstand entschärft werden.

Theoretisch wären auch noch retrograd (Abwinkelung >90 Grad) wirkende Instrumente denkbar, aber aufgrund des flachen Schnittwinkels und des sehr großen Überstandes ist diese Konstruktion nicht sinnvoll.

Das spezielle Konzept des Winkelkopfes ermöglicht besonders biegesteife Instrumente, die auch bei hoher Belastung ihre Position beibehalten können.

Der flexible Fräser 10 kann für den Biegebereich einen speziellen Abschnitt aufweisen, welcher einerseits die Bewegung der Instrumentenspitze erträgt und andererseits das Drehmoment weiterleiten kann. Dies kann beispielsweise ein dünner Draht, geflochtene Litzen oder ein Kardangelenk oder Ähnliches sein.

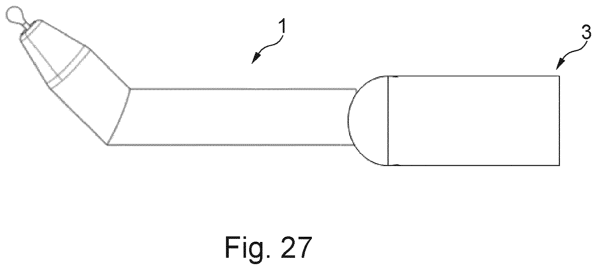

Fig. 27 zeigt den Schaft 1 mit einem medizinischen Handinstrument 3. Die Schäfte aller Ausführungsformen sind für das medizinische Handinstrument 3 geeignet und können mit dem medizinischen Handinstrument 3 verbunden werden.

### Bezugszeichenliste

- 1: Schaft
- 2: proximaler Schaftabschnitt
- 3: medizinisches Handinstrument
- 4: distaler Schaftabschnitt
- 6, 8: angestellte Stirnseite
- 10: flexibler Fräser
- 12: Außenrohr
- 14: Hohlrad
- 16: Innenverzahnung
- 18: Ritzel
- 20: Außenverzahnung
- 22: exzentrische Sicherungsbuchse
- 26: Wälzlager
- 28: Verstellbuchse
- 30, 60: Mitnahmezapfen
- 32: distale Schaftspitze
- 34: flexibles Übertragungselement
- 38: Kugeln
- 42: Federblech
- 48: Aufnahmenut

## Patentansprüche

1. Schaft (1)für ein medizinisches Handinstrument (3) mit einem distalen Schaftabschnitt (4) und einem proximalen Schaftabschnitt (2), deren jeweils einander zugewandte Stirnseiten (6, 8), von denen zumindest eine mit einem Anstellwinkel ungleich 90° bezüglich der jeweiligen Schaftlängsachse angestellt ist, sodass sich je nach Relativdrehposition der beiden Schaftabschnitte (2, 4) unterschiedliche Schaftformen ergeben,
**dadurch gekennzeichnet, dass**
ein Ritzel (18) des proximalen Schaftabschnitts (2) durch ein flexibles Übertragungselement (34; 42; 50; 52) rotationsübertragend mit einer Verstellbuchse (28) in dem distalen Schaftabschnitt (4) verbunden ist.

2. Schaft (1) nach Anspruch 1, **dadurch gekennzeichnet, dass** die Anstellwinkel gleich sind und sich je nach Relativdrehposition der beiden Schaftabschnitte (2,4) eine gerade oder eine abgewinkelte Schaftform ergibt.

3. Schaft (1) nach Anspruch 1 oder 2, **dadurch gekennzeichnet, dass** der proximale Schaftabschnitt (2) ein Hohlrad (14) mit einer Innenverzahnung (16) aufweist, die mit einer Außenverzahnung (20) des Ritzels (18) kämmt.

4. Schaft (1) nach einem der Ansprüche 1 bis 3, **dadurch gekennzeichnet, dass** die Verstellbuchse (28) einen Mitnahmezapfen (30; 60) aufweist, der formschlüssig mit einer distalen Schaftspitze (32) verbunden ist und die Rotation der Verstellbuchse (28) auf die distale Schaftspitze (32) überträgt.

5. Schaft (1) nach einem der Ansprüche 1 bis 4, **dadurch gekennzeichnet, dass** der proximale Schaftabschnitt (2) eine exzentrische Sicherungsbuchse (22) aufweist.

6. Schaft (1) nach einem der Ansprüche 1 bis 5, **dadurch gekennzeichnet, dass** das flexible Übertragungselement ein flexibles Federblech (42) mit einer seitlich angebrachten Kugel (44) ist.

7. Schaft (1) nach Anspruch 6, **dadurch gekennzeichnet, dass** die Kugel (44) des flexiblen Federblechs (42) in einer kugelförmigen Aufnahmenut (48) in dem Ritzel (18) aufgenommen ist und die der Kugel (44) gegenüberliegende Seite des Federblechs (42) mit der Verstellbuchse (28) verbunden ist.

8. Schaft (1) nach Anspruch 6, **dadurch gekennzeichnet, dass** die Kugel (44) des flexiblen Federblechs (42) in einer kugelförmigen Aufnahmenut (48) in der Verstellbuchse (28) aufgenommen ist und die der Kugel (44) gegenüberliegende Seite des flexiblen Federblechs (42) mit dem Ritzel (18) verbunden ist.

9. Schaft (1) nach einem der Ansprüche 1 bis 5, **dadurch gekennzeichnet, dass** das flexible Übertragungselement ein Silikonschlauch (34) ist.

10. Schaft (1) nach einem der Ansprüche 1 bis 5, **dadurch gekennzeichnet, dass** das flexible Übertragungselement ein flexibler Metallfaltenbalg (50) ist.

11. Schaft (1) nach einem der Ansprüche 1 bis 5, **dadurch gekennzeichnet, dass** das flexible Übertragungselement ein flexibles Metallrohr (52) ist.

12. Schaft (1) nach einem der Ansprüche 1 bis 11, **dadurch gekennzeichnet, dass** ein Abknickwinkel zwischen dem proximalen Schaftabschnitt (2) und dem distalen Schaftabschnitt (4) doppelt so groß ist, wie der Anstellwinkel der angestellten Stirnseiten (6, 8).

13. Schaft (1) nach einem der Ansprüche 1 bis 12, **dadurch gekennzeichnet, dass** der Abknickwinkel zwischen dem proximalen Schaftabschnitt (2) und dem distalen Schaftabschnitt (4) ein Maximum hat und der Anstellwinkel bei einer weiteren Rotation des distalen Schaftabschnitts (4) wieder kleiner wird.

14. Schaft (1) nach einem der Ansprüche 1 bis 13, **dadurch gekennzeichnet, dass** die Verstellbuchse (28) aus einem Gleitlagerwerkstoff, vorzugsweise PTFE oder POM, gefertigt ist und/oder eine Beschichtung mit PTFE aufweist.

## Claims

1. Shaft (1) for a medical hand instrument (3) comprising a distal shaft section (4) and a proximal shaft section (2), the respective end faces (6, 8) of which facing one another and of which at least one is set at an angle of incidence not equal to 90° with respect to the respective longitudinal axis of the shaft, so that different shaft shapes result depending on the relative rotational position of the two shaft sections (2, 4)
**characterized in that**
a pinion (18) of the proximal shaft section (2) is connected to an adjusting bush (28) in the distal shaft section (4) by a flexible transmission element (34; 42; 50; 52) in a rotationally transmitting manner.

2. The shaft (1) according to claim 1, **characterized in that** the angles of incidence are equal and that a straight or a bent shaft shape results depending on the relative rotational position of the two shaft sections (2, 4).

3. The shaft (1) according to claim 1 or 2, **characterized in that** the proximal shaft section (2) has a ring gear (14) with internal teeth (16) which meshes with external teeth (20) of the pinion (18).

4. The shaft (1) according to any of claims 1 to 3, **characterized in that** the adjusting bush (28) has a driving pin (30; 60) which is positively connected to a distal shaft tip (32) and transmits the rotation of the adjusting bush (28) to the distal shaft tip (32).

5. The shaft (1) according to any of claims 1 to 4, **characterized in that** the proximal shaft section (2) has an eccentric locking bush (22).

6. The shaft (1) according to any of claims 1 to 5, **characterized in that** the flexible transmission element is a flexible spring plate (42) with a laterally attached ball (44).

7. The shaft (1) according to claim 6, **characterized in that** the ball (44) of the flexible spring plate (42) is received in a spherical receiving groove (48) in the pinion (18) and the side of the spring plate (42) opposite the ball (44) is connected to the adjusting bush (28).

8. The shaft (1) according to claim 6, **characterized in that** the ball (44) of the flexible spring plate (42) is received in a spherical receiving groove (48) in the adjusting bush (28) and the side of the flexible spring plate (42) opposite the ball (44) is connected to the pinion (18).

9. The shaft (1) according to any of claims 1 to 5, **characterized in that** the flexible transmission element is a silicone hose (34).

10. The shaft (1) according to any of claims 1 to 5, **characterized in that** the flexible transmission element is a flexible metal gaiter (50).

11. The shaft (1) according to any of claims 1 to 5, **characterized in that** the flexible transmission element is a flexible metal tube (52).

12. The shaft (1) according to any of claims 1 to 11, **characterized in that** a bending angle between the proximal shaft section (2) and the distal shaft section (4) is twice as large as the angle of incidence of the angled end faces (6, 8).

13. The shaft (1) according to any of claims 1 to 12, **characterized in that** the bending angle between the proximal shaft section (2) and the distal shaft section (4) has a maximum and the angle of incidence becomes smaller again with a further rotation of the distal shaft section (4).

14. The shaft (1) according to any of claims 1 to 13, **characterized in that** the adjusting bush (28) is made of a sliding bearing material, preferably PTFE or POM, and/or has a coating with PTFE.

## Revendications

1. Tige (1) pour un instrument à main médical (3) avec une section de tige distale (4) et une section de tige proximale (2), leurs faces frontales (6, 8) respectivement tournées l'une vers l'autre, dont au moins une est ajustée selon un angle d'attaque différent de 90° par rapport à l'axe longitudinal de tige respectif, de sorte que différentes formes de tige en résultent selon la position de rotation relative des deux sections de tige (2, 4),
**caractérisée en ce que**
un pignon (18) de la section de tige proximale (2) est connecté par un élément de transmission flexible (34 ; 42 ; 50 ; 52) à une douille de réglage (28) dans la section de tige distale (4) en transmettant la rotation.

2. Tige (1) selon la revendication 1, **caractérisée en ce que** les angles d'attaque sont identiques et qu'une forme de tige droite ou coudée en résulte selon la position de rotation relative des deux sections de tige (2, 4).

3. Tige (1) selon la revendication 1 ou 2, **caractérisée en ce que** la section de tige proximale (2) présente une couronne (14) avec une denture intérieure (16) qui s'engrène avec une denture extérieure (20) du pignon (18).

4. Tige (1) selon l'une quelconque des revendications 1 à 3, **caractérisée en ce que** la douille de réglage (28) présente un tenon d'entraînement (30 ; 60) qui est connecté par complémentarité de formes à une pointe de tige distale (32) et transmet la rotation de la douille de réglage (28) à la pointe de tige distale (32).

5. Tige (1) selon l'une quelconque des revendications 1 à 4, **caractérisée en ce que** la section de tige proximale (2) présente une douille de sécurisation excentrique (22).

6. Tige (1) selon l'une quelconque des revendications 1 à 5, **caractérisée en ce que** l'élément de transmission flexible est une tôle à ressort flexible (42) avec une bille (44) placée latéralement.

7. Tige (1) selon la revendication 6, **caractérisée en ce que** la bille (44) de la tôle à ressort flexible (42) est reçue dans une rainure de réception sphérique (48) dans le pignon (18) et le côté de la tôle à ressort (42) opposé à la bille (44) est connecté à la douille de réglage (28).

8. Tige (1) selon la revendication 6, **caractérisée en ce que** la bille (44) de la tôle à ressort flexible (42) est reçue dans une rainure de réception sphérique (48) dans la douille de réglage (28) et le côté de la tôle à ressort flexible (42) opposé à la bille (44) est connecté au pignon (18).

9. Tige (1) selon l'une quelconque des revendications 1 à 5, **caractérisée en ce que** l'élément de transmission flexible est un tuyau en silicone (34).

10. Tige (1) selon l'une quelconque des revendications 1 à 5, **caractérisée en ce que** l'élément de transmission flexible est un soufflet métallique flexible (50).

11. Tige (1) selon l'une quelconque des revendications 1 à 5, **caractérisée en ce que** l'élément de transmission flexible est un tube métallique flexible (52).

12. Tige (1) selon l'une quelconque des revendications 1 à 11, **caractérisée en ce qu'**un angle de pliage entre la section de tige proximale (2) et la section de tige distale (4) est deux fois plus grand que l'angle d'attaque des faces frontales (6, 8) ajustées.

13. Tige (1) selon l'une quelconque des revendications 1 à 12, **caractérisée en ce que** l'angle de pliage entre la section de tige proximale (2) et la section de tige distale (4) présente un maximum et **en ce que** l'angle d'attaque redevient plus petit lors d'une rotation supplémentaire de la section de tige distale (4).

14. Tige (1) selon l'une quelconque des revendications 1 à 13, **caractérisée en ce que** la douille de réglage (28) est constituée d'un matériau pour palier lisse, de préférence du PTFE ou du POM, et/ou présente un revêtement avec du PTFE.
